# EUROPÄISCHE PATENTSCHRIFT

(11) **EP 1 595 557 B1**
(45) Veröffentlichungstag und Bekanntmachung des Hinweises auf die Patenterteilung: **30.04.2008**
(21) Anmeldenummer: 05013213.3
(22) Anmeldetag: 22.07.2003
(51) Int. Cl.: A61M 1/16

(54) **Intravenöser Oxygenator**
Intravenous oxygenator
Oxygénateur intraveineux

(30) Priorität: 22.07.2002 DE 10233290; 11.10.2002 DE 10247629
(43) Veröffentlichungstag der Anmeldung: 16.11.2005
(62) Teilanmeldung aus: 03787707.3
(73) Patentinhaber: NovaLung GmbH, 72379 Hechingen (DE)
(72) Erfinder: Cattaneo, Giorgio, Dipl.-Ing., 52064 Aachen (DE); Reul, Helmut, Prof., Dr.-Ing., verstorben (DE); Autschbach, Rüdiger, Prof. Dr. med., 52066 Aachen (DE)
(74) Vertreter: Castell, Klaus

(56) Entgegenhaltungen:
- US-A- 5 037 383

## Beschreibung

Die Erfindung betrifft einen intravenösen Oxygenator, insbesondere einen intravenösen Oxygenator mit integrierter Blutpumpe.

Im Bereich der klinischen Therapierung von Patienten mit beeinträchtigten Lungenfunktionen stellt sich regelmäßig die Aufgabe, die Lunge in ihrer Kernaufgabe, dem Anreichern des Bluts mit Sauerstoff durch Austausch gegen Kohlendioxid, zu unterstützen.

Das akute Lungenversagen ist weltweit eine der häufigsten Erkrankungen in der Intensivmedizin. Es äußert sich durch einen unzureichenden Austausch von Sauerstoff und Kohlendioxid und ist somit in höchstem Maße lebensbedrohlich und zieht enormen personellen wie finanziellen Aufwand bei der Behandlung nach sich. Trotz intensiver Forschung und neuartiger Therapien weist das akute Lungenversagen immer noch sehr hohe Mortalitätsraten von 50 bis 70 % auf.

Eine weitere Beeinträchtigung ergibt sich beispielsweise beim frühen Lungenversagen. Das frühe Lungenversagen ist eine der Hauptursachen der hohen Mortalität von Patienten, die ein Lungentransplantat erhalten haben. Im ersten Jahr nach einer Lungentransplantation stirbt etwa ein Viertel der Patienten.

Sowohl beim akuten Lungenversagen als auch beim frühen Lungenversagen gilt die maschinelle Beatmung als Standardtherapie. Mittlerweile ist es jedoch anerkannt, dass die maschinelle Beatmung erhebliche Schäden am Lungengewebe hinterlässt, da die erforderlichen hohen Beatmungsdrücke und -volumina zu einer Überblähung und somit zu einer mechanischen Zerstörung noch gesunder Lungenareale führen können.

Als Alternative wurde die extrakorporale Membranoxygenation entwickelt. Hier bedient man sich eines extrakorporalen Kreislaufs, in dem das Blut in einem künstlichen, aus Fasern bestehenden Oxygenator mit Sauerstoff angereichert und von Kohlendioxid entlastet wird. Das Blut wird einer großen Vene entnommen, von einer Pumpe durch den Oxygenator gefördert und wieder in die große Vene zurückgeführt.

Die Operation bei dieser Therapie ist leider sehr invasiv und das Blutungsrisiko entsprechend hoch. Außerdem fördert der intensive Kontakt des Bluts mit künstlichen Oberflächen die Thrombenbildung und führt zur Schädigung der Blutkörperchen.

Um hier Abhilfe zu schaffen, werden seit etwa 15 Jahren intravenöse, implantierbare Vorrichtungen zur Oxygenierung des Blutes untersucht. Eine solche Lösung führt zu einem äußerst geringen Kontakt zwischen Blut und künstlichen Oberflächen. Der Oxygenator wird bei dieser Therapie durch eine Femoralvene im Bein eingeführt und in die untere Hohlvene positioniert.

So offenbart die US 4,583,969 als älteste Schrift einen Membranoxygenator zur Positionierung in der Hohlvene. Der Oxygenator weist ein etwa 50 cm langes Bündel von 1.200 Hohlfasern auf. Sauerstoff wird durch die Hohlfasern geleitet, sodass durch reine Gasdiffusion Sauerstoff in das Blut und Kohlendioxid in die Hohlfaser übergeht. Das Faserbündel verursacht jedoch nachteilig einen hohen Strömungswiderstand, sodass sich das Blut in mehreren Punkten stauen und sich dort Thromben bilden können. Außerdem erfolgt nur ein sehr geringer Gasaustausch, was sich darauf zurückführen lässt, dass infolge der im Wesentlichen parallelen Anordnung der Fasern nur eine unzureichende Blutdurchmischung erfolgt.

Im Folgenden stützten sich alle weiteren Entwicklungen auf eine verbesserte Faser- und Strömungskonfiguration. Insbesondere wurde der Durchbruch in der Erhöhung der Blutgeschwindigkeit und der senkrechten Anströmung der Fasern gesehen. Die senkrechte Anströmung der Fasern und die starke Blutdurchmischung bringen jedoch einen hohen Strömungswiderstand mit sich.

Um dies zu kompensieren, schlägt die EP 0 507 724 A1 einen intravenösen Oxygenator vor, bei welchem die Fasern zwar längs der Vene liegen, in der Mitte der Fasern jedoch entlang der Längsachse des Oxygenators ein pulsierender Ballon angeordnet ist, welcher das Blut senkrecht zur Längsachse durch die Fasern drückt. Der Ballon nimmt jedoch relativ viel Platz in Anspruch und reduziert somit die Anzahl möglicher Fasern so stark, dass nur etwa ein Fünftel des erforderlichen Gasaustauschs erreicht wird.

Die US 5,037,383 schlägt einen intravenösen Oxygenator vor, bei welchem das Blut die Fasern bis auf kleine Randbereiche senkrecht und mit hoher Geschwindigkeit anströmt. Dies begünstigt zwar den Gasaustausch, verursacht aber andererseits sehr hohe Druckverluste mit bis über 100 mmHg Drucksäule.

Um das Problem des hohen Druckabfalls zu lösen, offenbart die US 5,814,011 einen Oxygenator, welcher in einer seitlich undurchlässigen Hülle die Gasaustauschfasern und eine Blutpumpe aufweist. Die Pumpe erzeugt ein lokales Druckgefälle innerhalb der Hülle, sodass das Blut mit hohem Druck durch die Fasern strömen und hierbei so viel Energie abbauen kann, dass es beim Austritt aus der Hülle schadlos wieder in die Vene eingeführt werden kann.

Auch mit diesem Vorschlag kann allerdings das erforderliche Maß an Gasaustausch bisher nicht dargestellt werden. Das dringende Bedürfnis nach einer besonders geeigneten Konstellation von Fasern und/oder Strömung bestand fort.

Die US 5,037,383 schlägt einen Oxygenator vor, bei welchem mehrere sequentiell angeordnete Faserbündel an Faserhaltern angeschlossen sind, wobei jeweils zwei Faserhalter über längselastische Gummielemente verbunden sind. Zum Einführen des Oxygenators in die Vene können sich die Gummielemente unter Belastung strecken, sodass sich die Fasern flach an den Oxygenator legen. Dies spart Platz beim Einführen. Weitere Oxygenatoren mit Hohlfasern sind beispielsweise aus der US 5,098,376 A, aus der EP 0 631 790 A2, US 4,850,958 A, aus der DE 90 02 100 U, aus der US 4,911,689 A, aus der US 5,125,902 A, aus der WO 02/076530 A1 oder aus der WO 97/39785 A1 bekannt.

Der Erfindung liegt die Aufgabe zu Grunde, dem Oxygenator im Betrieb in der Hohlvene eine gut geeignete räumliche Ausdehnung zu verleihen. Diese Aufgabe löst ein intravenöser Oxygenator zum Einführen in eine Vene, mit einem Faserbündel sauerstoff- und kohlendioxiddurchströmbarer Fasern, wobei die Fasern jeweils mit einem ersten Anschluss an eine Gaszufuhr angeschlossen sind und mit einem zweiten Anschluss an einen Gasabzug angeschlossen sind, sodass Sauerstoff und Kohlendioxid von den ersten Anschlüssen durch die Fasern zu den zweiten Anschlüssen strömen kann, wobei die Fasern mit einem ersten und einem zweiten Faserhalter verbunden und entlang einer Längsachse des Oxygenators verlagerbar sind, wobei die Faserhalter gegeneinander verdrehbar um die Längsachse des Oxygenators und vorzugsweise längs dieser Achse verlagerbar gelagert sind und wobei sich der Oxygenator weiterhin kennzeichnet durch ein radial verformbares Gehäuse mit einer undurchlässigen Hülle.

Ein radial verformbares Gehäuse ist insbesondere dann von Vorteil, wenn ein Oxygenator in eine besonders betriebstaugliche Position durch Tordieren von Hohlfasern gebracht werden soll. Beim Einführen in die Hohlvene kann der Oxygenator sehr kompakt in Form und Ausmaß sein. Sobald die Fasern jedoch tordiert werden und sich dadurch radial ausbreiten, nehmen sie das Gehäuse radial mit. Um den maximalen Durchmesser sicher festlegen zu können, kann das Gehäuse einen größten Durchmesser haben, welcher im voraus festgelegt ist. Die Erfindung wird detaillierter im Folgenden beschrieben (vgl. Seiten 6 ff.). Zunächst folgt jedoch schwerpunktmäßig die Beschreibung eines unabhängigen weiteren Oxygenators, bei dem zwei benachbarte Faserbündel gleichsinnig tordiert sind.

Die Fasern können dabei in radialer Erstreckung eine Form haben, welche in etwa der Form aus der US 5,037,383 bekannt ist. Dort verlaufen die Fasern in Schlaufen, welche von einem zentralen mit der Längsachse des Oxygenators angeordneten Katheter ausgehen. Die Schlaufen verlaufen vom Zentrum des Oxygenators nach außen, wo sie um etwa 180° gekrümmt sind, um umzukehren und wieder zurück zum Zentrum zu verlaufen. Am Ort der Umkehr darf die Krümmung jedoch nicht allzu stark sein, weil sonst die hohlen Fasern knicken und sich dadurch verschließen oder jedenfalls den Strömungswiderstand stark erhöhten können. Die Fasern verlaufen daher zumindest für einen kurzen Abschnitt etwa parallel zur Längsachse des Oxygenators.

Demgegenüber entsteht für die Fasern bei dem tordierten Faserbündel am Umkehrpunkt gar keine Gefahr, zu stark gekrümmt zu werden und sich deshalb zu verschließen. Vielmehr beginnen sich die Fasern bereits auf dem Weg nach außen in Umfangsrichtung zu krümmen und verlaufen am Umkehrpunkt für eine relativ lange Strecke im Wesentlichen entlang des Umfangs senkrecht zur Längs-achse des Oxygenators. Vorteilhaft wird somit zum einen eine zu enge Krümmung vermieden. Zum anderen liegen aber sogar am Umkehbereich die Fasern noch senkrecht zur Längsachse, also im Betrieb in cross-flow-Anordnung zur Blutströmung. Mithin ermöglicht es die Tordierung, die Fasern nahezu über den gesamten Verlauf zwischen den Anschlüssen - mit wenigen Millimetern Ausnahme unmittelbar an den Anschlüssen, wenn die Anschlüsse nicht senkrecht zur Längsachse liegen - im Wesentlichen senkrecht zur Längsachse ausbreiten zu können. Das Blut kann daher die Fasern nahezu über deren gesamte Länge in cross-flow-Anordnung anströmen, was zu einer weiteren Verbesserung der Gasaustauschwirkung führt.

Vorteilhaft kann die Tordierung so vorliegen, dass die Anschlüsse eine relative Verdrehung von 90° bis 300°, vorzugsweise von 150° bis 270°, besonders bevorzugt von etwa 240°, pro 35 mm laufender Faserlänge haben. Umfangreiche Versuche haben gezeigt, dass bei einer Tordierung in diesem Maße besonders gut Sauerstoff und Kohlendioxid ausgetauscht werden. Die zugrundeliegende Erkenntnis, dass eine Tordierung des Faserbündels zu einer Steigerung der Diffusion führt, gilt nur bis zu einem Schwellenwert der Tordierung. Darüber sinkt die Diffusion wieder.

Alternativ und kumulativ hierzu ist es von Vorteil, wenn zumindest eine Vielzahl, bevorzugt zumindest eine Mehrzahl der Fasern, im Faserverlauf zwischen den Anschlüssen einen Winkel von 30° bis 75°, bevorzugt von 42° bis 71 °, besonders etwa 62°, gegenüber der Längsachse bei Projektion der Längsachse und des Faserverlaufs auf einen mit der Längsachse koaxialen Projektionszylinder annehmen.

Bei einem tordierten Faserbündel liegen die Anschlüsse einer Faser um die oben angeführten Verdrehungswinkel um die Längsachse des Oxygenators verdreht. Die Fasern können auf vielfältige Weise an den Anschlüssen gelagert sein, beispielsweise frei drehbar oder auch fest eingespannt. Wenn die Fasern frei drehbar angeschlossen sind, wird sich bei einer Tordierung des Faserbündels eine Verdrehung der Lagerung so ergeben, dass sich die Fasern - abgesehen vom radialen Verlauf, also dem Verlauf der Entfernung zwischen Faser und Längsachse - im Wesentlichen direkt von Anschluss zu Anschluss erstrecken. Bei der Abwicklung des Mantels eines mit der Längsachse koaxialen Projektionszylinders ergeben sich die beiden Anschlusspunktprojektionen als unterschiedlich weit von der Längsachsenprojektionsgerade entfernt. Die direkte Erstreckung der Faser von Anschluss zu Anschluss äußert sich darin, dass die Faserprojektion zumindest im Wesentlichen eine Gerade ist.

Bei einer Einspannung der Fasern an den Anschlüssen kann insbesondere eine solche Einspannung in Betracht kommen, bei welcher die Faserenden parallel zur Längsachse gelagert sind. Bei der Tordierung eines Bündels solcher Fasern ergibt sich in der Projektion des Faserverlaufs keine Gerade, sondern im Wesentlichen eine punktsymmetrische Kurve mit zwei gegenläufigen Krümmungen. Am ersten Anschluss verläuft die Faser aufgrund ihrer Einspannung im Räumlichen wie in der Projektion parallel zur Längsachse. Sie nimmt sofort eine Krümmung an, welche im Räumlichen gleichsinnig mit der Verdrehung des gegenüber liegenden Anschlusses ist. Bei der Projektion äußert sich dies als Krümmung zur Längsachsenprojektion hin oder von dieser weg, je nachdem, ob die Projektion des gegenüberliegenden Anschlusses näher an der Längsachsenprojektion liegt als die Projektion des ausgehenden Anschlusses oder weiter entfernt. Etwa in der Mitte hat der Faserverlauf einen Wendepunkt und verläuft im Weiteren gegensinnig gekrümmt zum gegenüberliegenden Anschluss, bis der Verlauf genau dort ankommend wieder parallel zur Längsachse ist.

Die vorgeschlagenen Winkel zwischen Faserprojektion und Längsachsenprojektion ergeben gemäß der Auswertung aufwendiger Versuche sehr gute Diffusionsergebnisse von Sauerstoff und von Kohlendioxid in die Faser, insbesondere dann, wenn die Fasern über einen Großteil ihrer Länge im genannten Winkelbereich liegen. Sie weichen überraschend stark von der bisher effektivsten Faserkonfiguration ab, derzufolge die Fasern einfach radial von der Längsachse des Oxygenators abstehen und dadurch rechtwinklig angeströmt werden. Es sei darauf hingewiesen, dass eine rechtwinklige Anströmung auch bei der vorliegend vorgeschlagenen Konfiguration einfach dadurch erreicht werden kann, dass die Fasern im radialen Verlauf - welcher sich nicht in der Zylinderprojektion widerspiegelt - eine großwinklige Ausrichtung zur Längsachse annehmen. Beispielsweise können sich die Fasern in ihrem radialen Verlauf zunächst stark vom Oxygenator nach außen wegkrümmen, einen Winkel von etwa 90° annehmen und bis zu einem gewissen, nicht zu kurzen Abstand vor der Hälfte der Faserlänge diesen Winkel haltend nach außen verlaufen. An der Hälfte der Faserlänge kann ein Umkehrbereich mit einer Krümmung von insgesamt etwa 180° vorliegen, welche die Faser wieder rechtwinklig zurück nach innen, also auf die Längsachse des Oxygenators zu, verlaufen lässt. Dabei kann die Faser im Umkehrbereich - wie oben erläutert - insbesondere etwa mit dem Umfang des Faserbündels senkrecht zur Längsachse des Oxygenators verlaufen, um auch den Faserabschnitt im Umkehrbereich zur cross-flow-Anströmung zur Verfügung stellen zu können. Kurz vor dem gegenüberliegenden Anschluss wäre die Faser wieder um etwa 90° gekrümmt, um parallel zur Längsachse in die Anschlusslagerung zu stoßen. Eine solche Anordnung führt vorteilhaft zu einer überwiegend rechtwinkligen Anströmung der Fasern bei gleichzeitiger Tordierung des Faserbündels.

Unabhängig vom genauen Verlauf der Fasern wird vorgeschlagen, dass das Faserbündel an einer undurchlässigen Hülle anliegt. Eine undurchlässige Hülle um das Faserbündel bewirkt eine starke Kanalisierung des Blutstroms und kann somit das Blut durch die Fasern zwingen. Wenn sich zwischen dem Faserbündel und der Hülle ein Spalt ergibt, verteilt sich der Blutstrom entsprechend den Strömungswiderständen auf den gesamten durchströmten Raum, also fließt wenig Blut mit geringer Geschwindigkeit durch die Fasern, während viel Blut mit hoher Geschwindigkeit und ohne die Möglichkeit des Gasaustauschs mit den Fasern außen an den Fasern vorbeiströmt. Dies wird vermieden, wenn die Hülle unmittelbar außen um das Faserbündel herum verläuft. Hierzu kann die Hülle insbesondere elastisch sein, sodass sie sich automatisch bis zum Kraftschluss mit dem Faserbündel zusammenzieht beziehungsweise der Ausdehnung des Oxygenators folgt.

Um möglichst viel Blut aufnehmen zu können, ist es von Vorteil, das tordierte Faserbündel möglichst groß zu dimensionieren. Es ist jedoch gefährlich, mit dem Faserbündel die gesamte Hohlvene auszufüllen, denn bei Verstopfen des Faserbündels oder bei Ausfall der Blutpumpe wäre dann eine Blutzirkulation kaum noch möglich. In einer vorteilhaften Ausführungsform hat das tordierte Faserbündel daher einen Durchmesser von 15 bis 30 mm, bevorzugt einen Durchmesser von 15 bis 25 mm. Nach Literaturangaben hat die menschliche vena cava einen Durchmesser von etwa 30 mm. Genauere Untersuchungen des Anmelders haben jedoch nennenswert kleinere Durchmesser ergeben. Daher wäre bei den bekannten Oxygenatoren mit einer vollständigen Ausfüllung der Hohlvene zu rechnen. Der hier beschriebene Oxygenator kann hiervon abweichen. Zum einen ist mit der vorgeschlagenen Faserkonfiguration der Gasaustausch so effektiv, dass auch mit reduziertem Querschnitt gegenüber bisherigen Oxygenatoren höhere Gasaustauschwerte erreicht werden. Zum anderen bewirkt eine gewisse Umströmbarkeit des Oxygenators aber vorteilhaft auch, dass seitlich vom Oxygenator in die Hohlvene eintretendes Blut gegen die eigentliche Blutströmungsrichtung außen am Mantel des Oxygenators entlang zu dessen Eingang strömen kann. So kann je nach Länge und genauer Positionierung des Oxygenators beispielsweise Blut von der Leber bezüglich der eigentlichen Stromrichtung stromabwärts des Oxygenatoreingangs in die Hohlvene eingeleitet werden. Bei ausreichender Umströmbarkeit kann dieses zumindest teilweise der Strömung folgen, welche stromaufwärts in den Oxygenator eintritt. Somit kann ein erhöhter Blutstrom den Oxygenator durchlaufen.

Zur genauen Begrenzung des Oxygenators kann ein radial verformbares Gehäuse vorgesehen sein. Auf dieses kann auch die undurchlässige Hülle aufgebracht sein. Um den maximalen Durchmesser sicher festlegen zu können, wird vorgeschlagen, dass das Gehäuse einen größten Durchmesser von höchstens 30 mm, insbesondere von höchstens 15 bis 25 mm, annehmen kann. Hierfür kann konstruktiv beispielsweise dadurch Sorge getragen werden, dass in die Struktur des Gehäuses ein umlaufendes Zugband mit einer auf den größten Durchmesser abgestimmten Länge hat. Das Zugband sollte möglichst unelastisch sein, während das Gehäuse und/oder die Hülle insbesondere quer zur Längsachse sehr elastisch sein können. Ein Ausdehnen des Gehäuses führt zu einer Straffung des Zugbands, bis dieses einer weiteren Ausdehnung des Umfangs nicht mehr stattgibt. Sofern das Volumen des Gehäuses weiter steigt, führt eine unelastische Umfangsbegrenzung sogar dazu, dass das Gehäuse am Zugband im Querschnitt eine Kreisform annimmt, denn bei der Kreisform ist das Verhältnis von Fläche zu Umfang maximal. Ein Gehäuse mit integriertem Zugband kann besonders einfach durch ein Drahtgitter dargestellt werden.

Alternativ hierzu kann die undurchlässige Hülle auch so gestaltet sein, dass sie ohne externe Umfangsbegrenzer einer Aufweitung nur bis zu einer vorbestimmten Grenze stattgibt. Wenn im Oxygenator über eine Pumpe ein gegenüber dem Blut höherer Druck erzeugt wird, reicht eine solche Hülle bereits als Gehäuse des Oxygenators aus. Durch den Überdruck weitet sich die Hülle ins Blut aus, bis die aus der Druckdifferenz stammende Aufweitungskraft durch Hinzukommen einer hüllenimmanenten Verkleinerungskraft - beispielsweise eine elastische Tangentialzugkraft - ausgeglichen ist und die Hülle in einem stabilen Kräftegleichgewicht zur Ruhe kommt. Als Material für eine solche Hülle können sich beispielsweise Polyurethan oder Silikon gut eignen.

Alternativ und kumulativ zum Vorgenannten wird vorgeschlagen, dass die Anschlüsse des tordierten Faserbündels gegen selbständiges Entdrehen gesichert sind. Die tordierte Konstellation soll zum Betrieb des Oxygenators möglichst konstant aufrechterhalten bleiben. Es können jedoch Kräfte auf das Faserbündel einwirken, welche eine Rückdrehung der Torsion antreiben. Die Kräfte können externen Ursprungs sein - beispielsweise induziert durch Reibung an der Venenwand oder durch die Impulskraft der Blutströmung, wenn sie von einer Strombahn längs der Vene am Faserbündel zu einer rotierenden Sekundärströmung umgeleitet wird. Die Kräfte können aber auch im Oxygenator entstehen, beispielsweise durch eine Rückstellkraft der Fasern, wenn diese an den Anschlüssen eingespannt sind und im Ruhezustand eine andere Form haben als im gewundenen Zustand. Durch eine Sicherung gegen Entdrehen wird sichergestellt, dass das Faserbündel nicht ohne bewusstes menschliches Zutun die tordierte Konfiguration verlässt.

Dabei ist es von Vorteil, wenn das Faserbündel nur bis zu einer Grenzkraft gegen Entdrehen gesichert ist. Durch eine geeignete Verbindung nach außen können auch in Betriebslage des Oxygenators auf die Anschlüsse gegenläufige Momente aufgebracht werden, beispielsweise durch gegenläufiges Verdrehen zweier ineinander befindlicher Katheter, wobei der äußere Katheter mit einem Anschluss und der innere Katheter mit dem anderen Anschluss momentenschlüssig ist. So können selbst im Betrieb Momente auf den Oxygenator aufgebracht werden, die im Wesentlichen nur durch die Torsionsfestigkeit der Katheter begrenzt werden. Eine Drehsicherung kann deshalb bevorzugt so ausgeführt sein, dass sie bei Überschreiten einer Grenzkraft, nämlich vorzugsweise einer so hohen Grenzkraft, dass sie aller Voraussicht nach nur bei bewusstem Verdrehen der Anschlüsse auftritt, der Verdrehung nachgibt. Der Begriff Grenzkraft sei dabei im Sinngehalt mit dem Begriff Grenzmoment gleichgesetzt, denn über die Grenzkraft definiert sich das Grenzmoment als Produkt mit einem Krafthebel der Grenzkraft.

Alternativ und kumulativ ist es von Vorteil, wenn Mittel zur Begrenzung einer weitergehenden Verdrehung der Anschlüsse des tordierten Faserbündels gegeneinander vorgesehen sind. Ebenso wie Kräfte zum Rückdrehen des tordierten Faserbündels können auch interne und externe Kräfte zum weitergehenden Verdrehen der Anschlüsse auftreten. Zwar begrenzen die Fasern selbst die mögliche Verdrehung der Anschlüsse, denn bei einer vollständigen - um den Oxygenator verlaufenden - Streckung der Fasern kann ein weiteres Verdrehen nur nach Reißen der Fasern erfolgen; vorschlagsweise sind aber Mittel vorgesehen, welche die weitere Verdrehung bereits vor Streckung der Fasern stoppen oder wie oben zur Rückdrehung erläutert bis zu einer Grenzkraft behindern. Es ist von besonderem Vorteil, wenn das Faserbündel in der optimalen Konstellation zu beiden Richtungen zumindest bis zu einer Grenzkraft gesichert ist.

Eine Sicherung der beschriebenen Art kann konstruktiv besonders gut erreicht werden über einen Kraftschluss eines ersten Faserhalters mit einem zweiten Faserhalter, wobei die Faserhalter mit den Anschlüssen verbunden sind. Die Aufgabe, die Anschlüsse zu sichern, wird somit transformiert zu der Aufgabe, die Faserhalter gegen Verdrehung zu sichern, wofür bei geeigneter Gestaltung der Faserhalter beispielsweise mehr Platz zur Verfügung stehen kann als an den Anschlüssen.

Gemäß einer vorteilhaften Ausführungsform sind die Faserhalter im Innenraum des Faserbündels oder der Faserbündel angeordnet. Sie können insbesondere zylindermantelförmige Gleitkörper sein, welche einen zentralen Katheter umgeben, und auf dem Katheter verschieblich entlang der Längsachse des Oxygenators sein, welche zweckmäßigerweise durch den zentralen Katheter gebildet sein kann. Bei Anordnung eines zylindermantelförmigen Gleitkörpers direkt innen an einem Faseranschluss ist der Faserverlauf durch den Faserhalter nicht beeinflusst. Die Halterung sorgt dann lediglich für eine radiale Fixierung des Anschlusses. Beispielsweise können die Faserenden am Fasermantel vergossen sein und mit ihren Stirnflächen an die Gaszufuhr beziehungsweise die Gasabfuhr angeschlossen sein, wobei die Gaszufuhr und die Gasabfuhr jeweils am Anschluss eine hohlringförmige Kammer bereitstellen. Diese Kammer kann auf der Ringinnenseite einfach mit dem Faserhalter mechanisch verbunden sein, beispielsweise durch Verklebung. Ein zylindermantelförmiger Faserhalter ist zudem besonders frei um die zentrale Längsachse insbesondere auch gegenüber einem benachbarten Faserhalter desselben Faserbündels - also demjenigen Faserhalter, welcher das gegenüberliegende Ende der Fasern oder deren Anschluss hält - drehbar.

Es versteht sich, dass eine Kammer der vorgeschlagenen Art nicht ein eigenständiges Bauelement sein muss. Vielmehr kann die Kammer beispielsweise auch durch einen Freiraum zwischen zwei Faseranschlüssen verkörpert sein. In diesem Fall wird vorgeschlagen, diesen Freiraum nach außen mit einer insbesondere zylindermantelförmigen Hülse dichtend zu begrenzen. Die Fasern zweier benachbarter Faserbündel können so weit gegenläufig in die Hülse eingeführt sein, dass zwischen den Stirnenden der Fasern ein Hohlraum verbleibt. Dieser kann geeignet an die Gaszufuhr und/oder an die Gasabfuhr angeschlossen werden.

Ein Oxygenator mit Faseranschlüssen, die einzeln mit Faserhaltern verbunden sind, welche gegeneinander verdrehbar um die Längsachse des Oxygenators gelagert sind, ist auch für sich betrachtet vorteilhaft.

In einer bevorzugten Ausführungsform weist der vorgeschlagene Oxygenator eine im Wesentlichen oder sogar ausgeprägt elastische Verbindung zwischen zwei in Längsrichtung des Oxygenators benachbarten Faserhaltern desselben Faserbündels auf. Vorteilhaft wird so bei Verformung des Oxygenators automatisch Energie zum Rückstellen der Verformung in der elastischen Verbindung gespeichert. Insbesondere sei an eine Verbindung gedacht, welche eine relative Verdrehung der Faserhalter durch Aufbringen einer Kraft beziehungsweise eines Moments unter zunehmender Gegenkraft beziehungsweise unter zunehmendem Gegenmoment zulässt.

Die aufgebrachte Kraft beziehungsweise das aufgebrachte Moment kann durch Verdrehung zweier Katheter - wie oben beschrieben - erfolgen, um die elastisch verbundenen Faserhalter aus einem Ruhezustand, in welchem das Faserbündel nicht tordiert ist, in den tordierten Einsatzzustand zu bringen. In diesem Fall hätte der Chirurg den Vorteil, dass der Oxygenator beim Einführen in die Hohlvene und beim späteren Entfernen keine Tendenz hat, sich zu verdrehen. Erst am Einsatzort würde die Verdrehung durch eine Handlung des Chirurgen erfolgen und fixiert werden. Zum Entfernen müsste der Chirurg lediglich die Fixierung abnehmen, und der Oxygenator würde seine untordierte Form annehmen.

Alternativ hierzu kann der Ruhezustand des Faserbündels in der tordierten Form liegen. In diesem Fall müsste der Chirurg zwar während des Einführens und während des Entfernens die Verdrehung unterbinden - beispielsweise durch Verklammern der zwei Katheter -, im Einsatz wäre das Faserbündel hingegen auch ohne äußere Kraft immer tordiert und aufgespannt. Der Ruhezustand kann dadurch gekennzeichnet sein, dass zwischen den Fasern und kraftübertragenden Verbindungen zwischen den Anschlüssen ein Momentengleichgewicht herrscht.

Für den Fall eines Ruhezustands im tordierten Zustand wird vorgeschlagen, dass der Ruhezustand bei einer relativen Verdrehung von 90° bis 300°, vorzugsweise von 150° bis 270°, besonders bevorzugt von etwa 240°, pro 35 mm Faserlänge, zwischen den beiden Anschlüssen liegt. Dies sind die bereits ausführlich erläuterten Werte, bei welchen der Gasaustausch überraschend stark abläuft.

Eine elastische Verbindung zwischen zwei Faserhaltern desselben Faserbündels - beziehungsweise zwischen zwei benachbarten Anschlüssen allgemein, falls mehrere Faserbündel nacheinander entlang des Oxygenators vorgesehen sind - kann mit besonderer Eignung eine Membran und/oder eine Linearfeder aufweisen. Eine Membran erlaubt es auf besonders einfache Weise, Momente zwischen den Faserhaltern desselben Faserbündels zu übertragen. Wenn die Membran als geschlossener Zylindermantel den Abstand zwischen zwei benachbarten ebenfalls zylindermantelförmigen Faserhaltern überbrückt, dann ergibt sich außerdem eine den Innenraum abdichtende Struktur gegenüber dem Außenraum, in welchem das Blut auf die Fasern trifft. Hierdurch kann die Struktur aus Faserhaltern und Verbindungsmembranen zur Gaszufuhr oder Gasabfuhr benutzt werden, was im Längsverlauf des Oxygenators einen Katheter überflüssig macht, wodurch der Oxygenator kompakter und kostengünstiger bauen kann.

Es sei darauf hingewiesen, dass ein Oxygenator mit einer elastischen Membranverbindung zwischen den Faserhaltern - insbesondere wenn durch die Verbindung ein abgedichteter Innenraum entsteht - auch unabhängig von den restlichen vorteilhaften Merkmalen vorteilhaft ist.

Eine Linearfeder kann insbesondere mit einem gegenüber einer Membran unterschiedlichen Ruhezustand vorgesehen sein, um den Membrankräften entgegen zu wirken und somit per Saldo die elastischen Kräfte innerhalb des Oxygenators zu reduzieren. Selbstverständlich kann eine Linearfeder auch unabhängig von einer Membran vorteilhaft zum Einsatz kommen. Insbesondere kann eine Linearfeder sogar parallel zur Längsachse zwischen zwei Faserhaltern angeordnet sein und die Faserhalter auseinander drücken, damit auf dem zentralen Katheter eine Zugkraft angreift. Hierdurch ist der zentrale Katheter bestmöglich vor Knicken geschützt. Ebenso gut kann eine Linearfeder oder eine gekrümmte Feder auch dazu dienen, die Faserhalter gegeneinander zu verdrehen. Hierdurch kann beispielsweise bei gleichzeitigem Vorhandensein einer Membran diese immer in leicht gespanntem Zustand gehalten werden. Wenn die Membran einen schlaffen Zustand annimmt, können Falten entstehen, an denen sich Blut stauen kann, was zu einer erhöhten Gefahr von Blutgerinnseln führt.

Wenn der Oxygenator mehrere in Reihe angeordnete Faserbündel hat, wird vorgeschlagen, dass diese gleichsinnig tordiert sind. Das Blut übt eine Kraft auf die ausgebreiteten Fasern aus und verformt somit die Faserlage. Abhängig von der Turbulenz und der Homogenität der Strömung können sich Bereiche ausbilden, in welchen die Fasern beispielsweise zu dicht liegen und kaum noch durchströmt werden. Angesichts dieses Problems kann es sinnvoll sein, wenn die Fasern zwischen zwei befestigten Faserstellen, beispielsweise den Anschlüssen, nur eine möglichst geringe Länge haben. Die Konstellation von mehreren in Reihe liegenden, gleichsinnig tordierten Faserbündeln hat den Vorteil, dass eine besonders gute Strömung entlang von Fasern über eine lange Fließstrecke des Bluts erzeugt wird, ohne dass die einzelnen Fasern zu instabil werden oder so lang werden, dass das in ihnen strömende Gasgemisch aus Sauerstoff und Kohlendioxid zu reich an Kohlendioxid wird. Bei mehreren Faserbündeln mit demzufolge auch mehreren Anschlusspaaren kann an jedem ersten Anschluss eine Zufuhr von Sauerstoff erfolgen. Es lässt sich ein stabiles Gerüst aus sehr vielen Fasern bereitstellen, was vorteilhaft mit einer großen Gesamtfaseroberfläche einhergeht.

Eine Anordnung von mehreren Faserbündeln auf separaten Faserhaltern wird durch elastische Verbindungen besonders begünstigt, denn beim Verdrehen eines Faserbündels wird ein Drehmoment auch auf die benachbarten Faserbündel übertragen. Hierdurch ergibt sich ohne weiteres Zutun eine gleichmäßige Drehmomentenverteilung und somit eine gleiche Tordierung aller Faserbündel, welche miteinander dergestalt verbunden sind.

Auch unabhängig vom Vorgenannten vorteilhaft ist ein Oxygenator mit gegeneinander verdrehbaren Faserhaltern, welcher sich auszeichnet durch einen ersten Mitnehmer am ersten Faserhalter und einen zweiten Mitnehmer am zweiten Faserhalter, wobei die Faserhalter zueinander gerichtet sind und bei koxialer Anordnung und Presskontakt der beiden Faserhalter gegeneinander eine relative Verdrehung des ersten Faserhalters gegenüber dem zweiten Faserhalter zumindest in eine Drehrichtung nur bis zu einer Drehgrenze ohne Mitnahme des zweiten Faserhalters zulassen. Wenn zwei aufeinander abgestimmte Mitnehmer dieser Art vorgesehen sind, fällt es dem Chirurgen besonders leicht, die erfindungsgemäße Tordierung des Faserbündels - insbesondere sogar eines Faserbündels, welches im nicht tordierten Zustand seinen Ruhezustand hat - auch ohne Sicht des Oxygenators zu finden. Bei aktiver Verdrehung des Faserbündels kann beispielsweise das benötigte Moment zunächst nicht wesentlich oder zumindest nur etwa linear anwachsen. Sobald die Drehgrenze erreicht ist, wirken jedoch die Mitnehmer an den zueinander gerichteten Seiten zusammen und geben eine haptische Rückkopplung zum Chirurgen, dass die gewünschte Tordierung ihrem Maße nach erreicht ist. Vorzugsweise können zwei Faserhalter benachbarter Faserbündel ohne Spiel verbunden sein.

Mit Blick auf den identifizierten Verdrehungszustand zum optimalen Gasaustausch sollte die Drehgrenze bei einer relativen Verdrehung von 90° bis 300°, vorzugsweise von 150° bis 270°, besonders bevorzugt bei etwa 240°, pro 35 mm Faserlänge, liegen.

Auch in Längsrichtung wird eine Anschlageinrichtung an den Faserhaltern zur Begrenzung einer Verlagerung der Anschlüsse zueinander vorgeschlagen. Eine solche Anschlageinrichtung kann beispielsweise direkt durch die Faserhalter verkörpert werden, sofern diese weit genug unter den Anschlüssen hervorragen, um ein Aneinanderstoßen zweier benachbarter Faseranschlüsse oder ein Verknicken der Fasern beim Ausbreiten der Fasern durch Zusammenrücken der Anschlüsse entlang der Längsachse zu verhindern.

Um beim Zusammenrücken der Anschlüsse auf einfache Weise und in genau vorgebbarem Maße die Faserbündel tordieren zu können, kann auch unabhängig vom Vorgenannten eine Spiralführung der Anschlüsse und/oder der Faserhalter vorgesehen sein. Die Spiralführung kann insbesondere an dem zentralen Katheter angeordnet sein und beispielsweise mit den Faserhaltern als Gewinde mit großer Gewindesteighöhe zusammenwirken.

In einer bevorzugten Ausführungsform weist der Oxygenator ein mit einem Faserbündel verbundenes Getriebe auf. Ein solches Getriebe kann beispielsweise den Verdrehungswinkel eines Anschlusses anzeigen, insbesondere des ersten Faserbündels, wenn mehrere Faserbündel seriell angeordnet sind. Der Chirurg kann somit objektiv erkennen, um welchen Winkel er den ersten Anschluss bereits verdreht hat.

Insbesondere wird vorgeschlagen, dass an einer Dreheinrichtung zum Tordieren mehrerer serieller Faserbündel ein Getriebe zwischen der Dreheinrichtung und einem Faserbündel so vorgesehen ist, dass das Getriebe eine Verdrehung der Dreheinrichtung - beispielsweise eines Drehrads - auf die Faserbündel mit einer Übersetzung überträgt, welche der Anzahl der Faserbündel oder dem Verhältnis der gesamten Länge der Faserbündel zu einer Normierungslänge entspricht. Beispielsweise können im Oxygenator zehn bis fünfzehn Faserbündel mit einer jeweils gleichen Faserlänge von 35 mm vorliegen. Sofern die Übersetzung des Getriebes der Anzahl der Faserbündel entspricht und die benachbarten Faserbündel geeignet miteinander verbunden sind, würden alle Faserbündel um denjenigen Drehwinkel tordiert, der an der Dreheinrichtung nur einmal gedreht worden ist. Beispielsweise überträgt ein Getriebe mit einer Übersetzung von 10:1 eine Drehung der Dreheinrichtung von 240° so auf den verbundenen Anschluss, dass dieser um 2400° verdreht wird. Bei geeigneter Verbindung der Anschlüsse benachbarter Faserbündel - insbesondere wie erläutert über die Faserhalter - hat bei zehn Faserbündeln schließlich jedes Faserbündel eine Tordierung von etwa 240°. Somit kann der Chirurg sehr kontrolliert und mit einer nur geringen Eigenbewegung den Tordierungszustand besonders gut einstellen, auch ohne den Oxygenator bei der Tordierung zu sehen.

Es sei darauf hingewiesen, dass sämtliche als vorteilhaft beschriebenen Merkmale in beliebiger Alternation oder Kumulation und auch unabhängig von einer Tordierung des Faserbündels vorteilhaft sind.

Die Erfindung wird nachfolgend anhand von Ausftihrungsbeispielen unter Bezugnahme auf die Zeichnung weiter erläutert. Dabei können gleiche Bezugsziffern gleiche oder gleichartige Elemente bezeichnen. In der Zeichnung zeigen
- Figur 1: schematisch einen zusammengefalteten Oxygenator bei Einführung durch eine Femoralvene,
- Figur 2: schematisch den erfindungsgemäßen Oxygenator aus Figur 1 ausgebreitet in einer Hohlvene,
- Figur 3: schematisch einen Schnitt durch den Oxygenator gemäß Figur 2 mit Darstellung der Gasströmung,
- Figur 4: schematisch einen Schnitt durch einen alternativen Oxygenator mit in mehrere serielle Einheiten unterteilten Fasern,
- Figur 5: ein Detail der schematischen Darstellung aus Figur 4 mit Darstellung der Gasströmung,
- Figur 6: in einem Detailschnitt zwei benachbarte und verbundene Schlitten zum Halten der Fasern,
- Figur 7: in einer Draufsicht eine Anordnung mehrerer gegeneinander verdrehter Schlitten,
- Figur 8: schematisch einen Schnitt durch einen weiteren Oxygenator mit einer veränderten Gasführung,
- Figur 9: in einem Detail den weiteren Oxygenator aus Figur 8 mit gekennzeichneter Gasströmung,
- Figur 10: die Schlitten aus den Figuren 8 und 9 in einer Draufsicht gemäß dem Schnitt X-X in Figur 9,
- Figur 11: in einem Längsschnitt einen Oxygenator mit stromaufwärts angeordneter Pumpeneinheit und
- Figur 12: schematisch den Querschnitt XII-XII aus Figur 11.

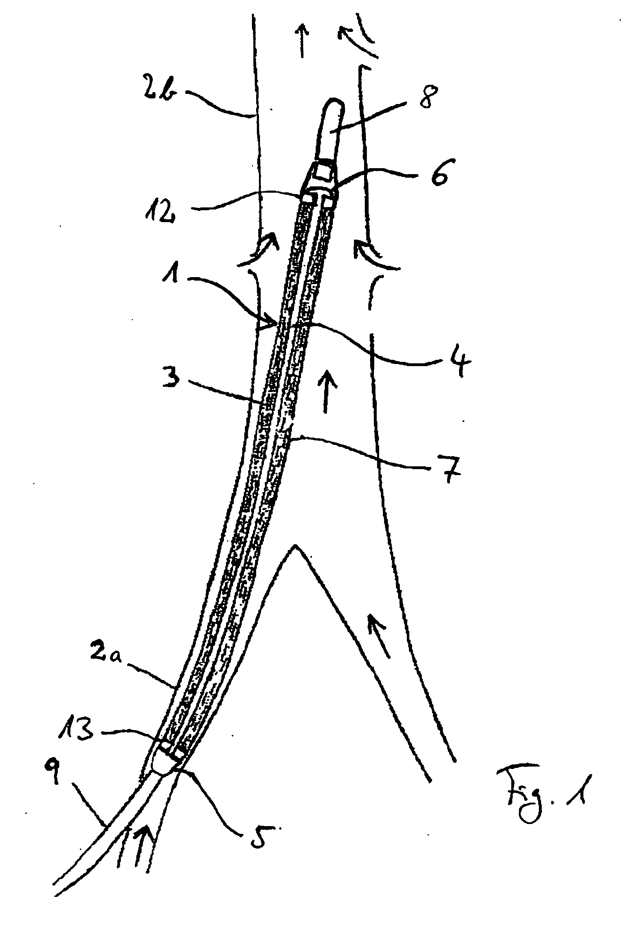

Der Oxygenator 1 in den Figuren 1 bis 3 wird durch die Femoralvene 2a eingeführt und in die Hohlvene 2b positioniert. Fasern 3 zum Gasaustausch sind während des Einsetzvorgangs aufgrund des beschränkten Insertionsraums zusammengefaltet und liegen längs eines zentralen Katheters 4. Der Durchmesser des Oxygenators ist in dieser Konfiguration sehr gering und der anatomischen Größe der Femoralvene 2a angepasst.

Der Katheter 4 ist von handelsüblicher Ausführung und hat die mechanischen Eigenschaften, die für medizinische Anwendungen vorausgesetzt werden. Das Faserbündel 3 ist an seinen Enden mit einer Zufuhrkammer 6 beziehungsweise einer Abzugkammer 5 verbunden, die das Faserbündel 3 zusammenhalten und gleichzeitig als Anschlüsse 12, 13 die Fasern 3 so aufnehmen, dass Gas von der Zufuhrkammer 6 durch den ersten Anschluss 12 durch die Fasern 3 und den zweiten Anschluss 13 zur Abzugkammer 5 strömen kann.

Ein Gehäuse 7 mit rundem Querschnitt umgibt das Faserbündel 3. Das zylindrische Gehäuse 7 hat als tragende Struktur ein verformbares Drahtgitter, welches einen maximalen Durchmesser annehmen kann, der geringfügig kleiner ist als der Durchmesser der Hohlvene 2b. Das Drahtgitter ist mit einer undurchlässigen elastischen Hülle verbunden, die der Verformung des Gitters folgt.

An einem Ende des Faserbündels 3 ist mit dem Gehäuse 7 und der Zufuhrkammer 6 eine Mikroaxialpumpe 8 verbunden. Ein Schlauch 9 ist mit dem Faserbündel 3 am anderen Ende verbunden. Der Schlauch 9 umgibt eine nach außen verlaufende Verlängerung (nicht sichtbar) des zentralen Katheters 4. Der Schlauch 9 ist gleichzeitig mit dem Gehäuse 7 nicht dichtend verbunden.

Die Fasern 3 sind an den Anschlüssen 12, 13 vergossen, haben aber freie Stirnflächen zur Verbindung mit den Kammern 5, 6. Der Katheter 4 verläuft zentral durch das Faserbündel 3 und ist pumpenseitig mit der Zufuhrkammer 6 verbunden. Die Zufuhrkammer ist wie geschildert mit dem Faserbündel 3 über den ersten Anschluss 12 verbunden. Am gegenüberliegenden Anschluss 13 sind die Fasern 3 mit der Abzugkammer 5 verbunden, welche ihrerseits an den neben der Katheterverlängerung verbleibenden Freiraum im Schlauch 9 angeschlossen ist. So ergibt sich ein Gasströmweg vom Katheter 4 über die Fasern 3 zurück zum Schlauch 9.

Der Schlauch 9 und der Katheter 4 laufen bis über die Einführungsstelle hinaus durch die Haut des Patienten aus dem Körper heraus.

Die Mikroaxialpumpe 8 ist seriell am Ende des Oxygenators 1 mit dem Gehäuse 7 und der Zufuhrkammer 6 verbunden. Die Pumpe 8 besteht im Wesentlichen aus einem Rotor 14, einem Motor 15 und einem Pumpengehäuse 16. Der Bluteinlass der Pumpe 8 befindet sich im Raum innerhalb der Hülle 7. Der Blutauslass befindet sich außerhalb der Hülle 7. Die Förderrichtung der Pumpe 8 ist gerichtet vom Anschluss 13 zum Anschluss 12, also in physiologischer Strömungsrichtung (durch einfache Pfeile gekennzeichnet).

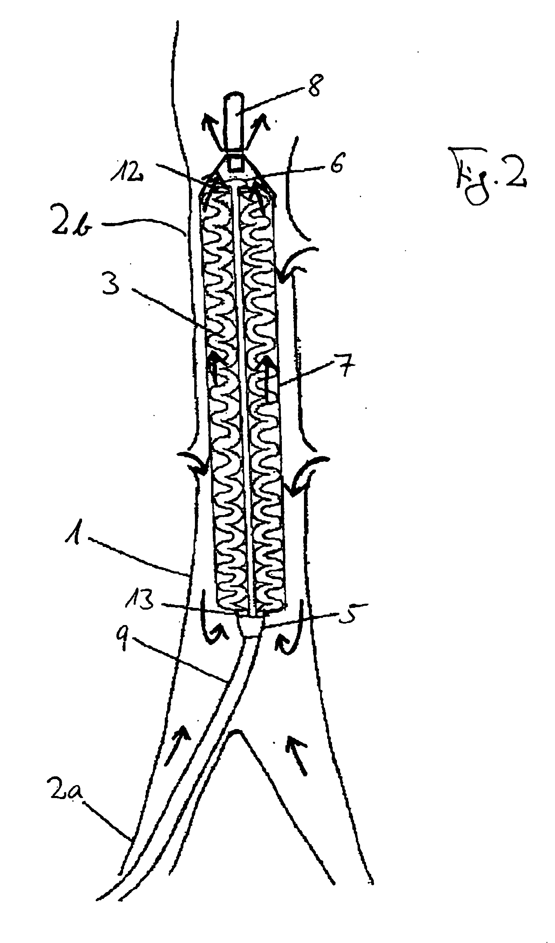

In der ausgebreiteten Konfiguration in Figur 2 liegt der Oxygenator in der unteren Hohlvene 2b. Das Faserbündel 3 ist an mehreren Stellen radial ausgebreitet und tordiert. Durch die Ausbreitung wird die Länge des Faserbündels 3 verkürzt und die umgebende Hülle 7 bis zum maximalen Durchmesser aufgeweitet. Das Faserbündel kann im gezeigten Beispiel insbesondere um 240° pro 35 mm langer Fasereinheit verdreht sein, wobei sich die Fasern in der Längserstreckung von einer ursprünglichen Länge von etwa 30 bis 35 mm auf etwa 14 mm verkürzt haben. Dabei kann das Faserbündel insbesondere 200 bis 250 Fasern mit einer gesamten Oberfläche von beispielsweise etwa 0,01 m² haben. Dies sind Konstellationen, bei welchen sich bei Versuchen ein sehr guter Gasaustausch ergeben hat.

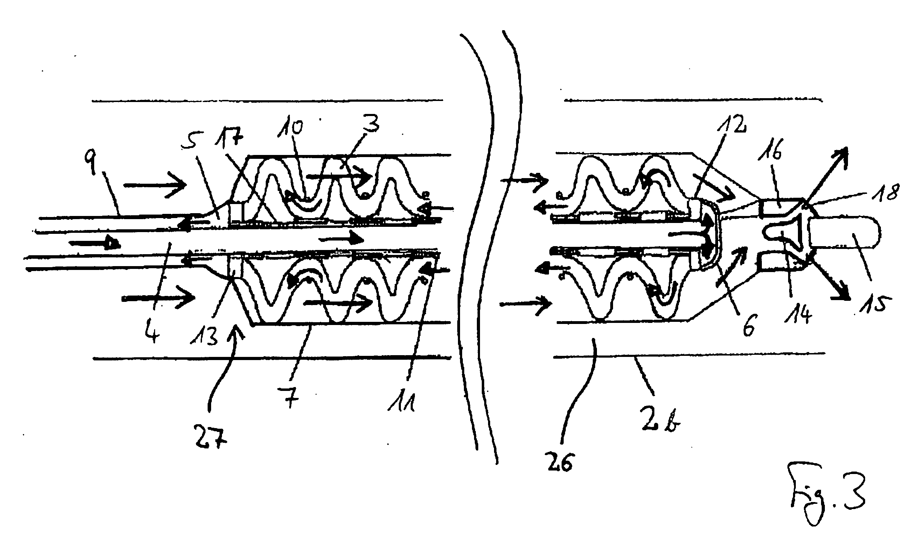

Das Faserbündel 3 des entfalteten Oxygenators 1 in Figur 3 ist in regelmäßigen Abständen mit dünnen Ringen 10 am Zentralkatheter 4 befestigt. Auf der Höhe der Ringe 10, zwischen den Fasern 3 und dem Katheter 4, befinden sich Führungsschlitten 11, welche die Fasern 3 halten. Durch Verschiebung des Schlauchs 9 über den Katheter 4 wird das Faserbündel längs komprimiert und somit ausgebreitet. Die Fasern 3 werden hierdurch gezwungen, sich in den Räumen zwischen zwei benachbarten Ringen 10 auszubreiten. Es bilden sich daher mehrere wellenförmige Fasereinheiten.

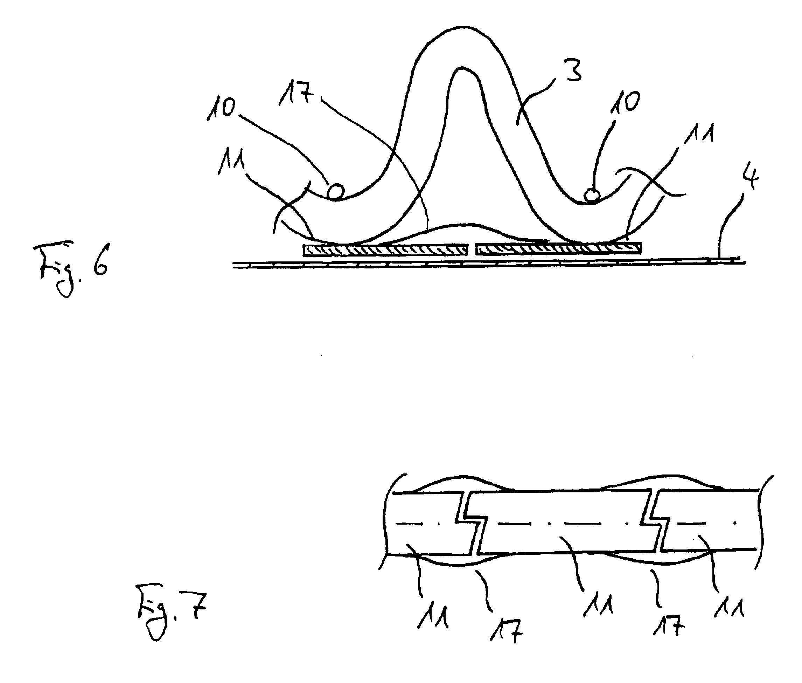

Die Schlitten 11 sorgen dafür, dass die Fasern während der Verschiebung des Schlauchs 9 und der damit einhergehenden Ausbreitung des Faserbündels 3 leicht auf dem zentralen Katheter gleiten können. Dabei haben die Schlitten 11 Stirnprofile, welche mit den jeweiligen Profilen der benachbarten Schlitten 11 zusammenwirken können (vgl. insbesondere Figur 6).

Das Faserbündel 3 kann bei der Komprimierung des Oxygenators 1 ausgebreitet werden, bis die Schlitten 11 mit ihren jeweiligen Nachbarschlitten 11 in Berührung stehen. Somit weisen alle Fasereinheiten zwischen zwei Ringen 10 am Schluss der Komprimierung die gleiche Länge auf - das Faserbündel 3 wird möglichst homogen ausgebreitet. Durch die Rotation des Schlauchs 9 wird das Faserbündel 3 tordiert. Das Profil der Schlitten 11 ist so ausgebildet, dass sich zwei benachbarte Schlitten 11 gegeneinander nur bis zu einem maximalen relativen Winkel verdrehen können, bevor durch zwei Mitnehmer an den beiden Schlitten 11 eine weitergehende relative Verdrehung verhindert wird. Wenn die maximale Drehung erreicht ist, haben die beiden Mitnehmer einen kraftschlüssigen Kontakt. Vorliegend wird dieser durch einen formschlüssigen Kontakt der beiden benachbarten Schlitten 11 gewährleistet. Somit hat jede Fasereinheit zwischen zwei Ringen 10 auch die gleiche Tordierung.

Der Raum zwischen zwei Schlitten 11 wird durch undurchlässige Membranen 17 abgedichtet. Außen sind die Fasern 3 zudem vom undurchlässig umhüllten Gehäuse 7 umgeben. Das Gehäuse 7 ist an einer Seite mit der Pumpe 8 dichtend und an der anderen Seite mit der Abzugkammer 5 nicht dichtend verbunden. Wenn das Gehäuse 7 an seinen Enden gedehnt wird, wird es länger und somit schlanker. Die Hülle folgt dabei der Bewegung des Gehäuses 7. Während des Einführens des Oxygenators 1 wird das Gehäuse 7 dadurch gedehnt, dass der Schlauch 9 und folglich die Abzugkammer 5 über den Katheter 4 von der Pumpe 8 fortgerichtet gezogen werden. Alternativ hierzu kann selbstverständlich auch auf eine Verbindung an der Seite der Kammer 5 verzichtet werden und das Gehäuse beziehungsweise die Hülle einfach zusammengefaltet werden, um den Oxygenator einzuführen.

Durch gegengerichtete Verschiebung des Schlauchs 9 nach Erreichen der Zielposition in der Hohlvene 2b, also in Richtung auf die Pumpe 8 zu, wird der Oxygenator 1 in seiner Längserstreckung komprimiert, sodass sich das Gehäuse 7 und die Hülle bis zum maximalen Durchmesser aufweiten. Nach Komprimierung und Tordierung füllen die Fasern 3 den gesamten Raum zwischen Katheter 4 und Gehäuse 7.

Die Fasern laufen im Ausführungsbeispiel des Oxygenators 1 über die gesamte Länge des Faserbündels 3 als durchgehende Gasleitungen. Durch den Katheter 4 wird im Betrieb des Oxygenators 1 Sauerstoff zugeführt. Der Sauerstoff strömt durch den Katheter 4 in die Zufuhrkammer 6 (Gasströmung ist gekennzeichnet durch geschlossene Pfeile). Von dort strömt der Sauerstoff über den ersten Anschluss 12 in die Fasern 3, an deren Oberfläche diffusiver Gasaustausch mit dem Blut stattfindet. Dabei geht Sauerstoff ins Blut über und tauscht sich dort gegen Kohlendioxid aus. Am zweiten Anschluss 13 befindet sich in den Fasern ein Gasgemisch aus Sauerstoff und Kohlendioxid. Das Gasgemisch strömt durch die Abzugkammer 5 in den Schlauch 9 und wird durch diesen aus dem Körper des Patienten herausgeführt.

Das Blut fließt in den Oxygenator 1 auf Höhe des Anschlusses 13, strömt das tordierte Faserbündel 3 innerhalb des Gehäuses 7 an und gelangt zu der Pumpe 8. Das Blut wird dort vom Rotor 14 in Fließrichtung der Vene 2a, 2b gefördert und verlässt den Oxygenator 1 durch einen Auslass 18.

Durch die Umlenkungsvorgänge beim Umströmen der Fasern 3 erfährt das Blut einen Verlust an Strömungsenergie. Daher ist der Blutdruck direkt an der Pumpe niedriger als am Oxygenatoreingang an der Seite des zweiten Anschlusses 13, wo ein physiologischer Druck herrscht. Der Druckabfall wird von der Pumpe 8 wieder ausgeglichen, sodass der Druck am Auslass 18 wieder den physiologischen Druck aufweist. Das Blut außerhalb des Gehäuses 7 erfährt aufgrund eines ausreichend großen Umströmungsraumes 26, eines strömungsgünstig geformten Vorderseitenprofils 27 und der geringen Strömungsrauheit außen am Gehäuse 7 keinen bedeutenden Druckverlust. Innerhalb des Gehäuses 7 herrscht daher ein geringerer Druck als in dem umgebenden Raum 26 in der Hohlvene 2b. Daher kann in der Hohlvene 2b ein physiologischer Druck gewährleistet bleiben, was die Organe vor Überdruckbelastung schont und eine physiologische Blutrückkehr zum Herzen ermöglicht.

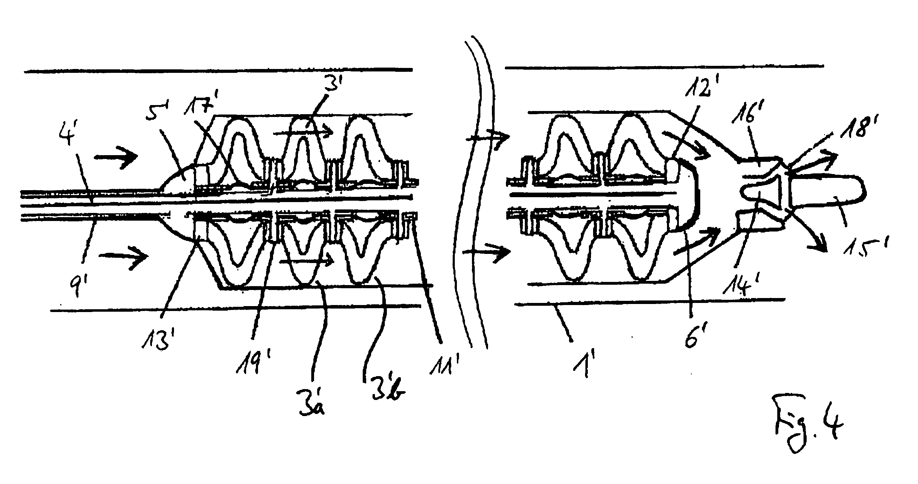

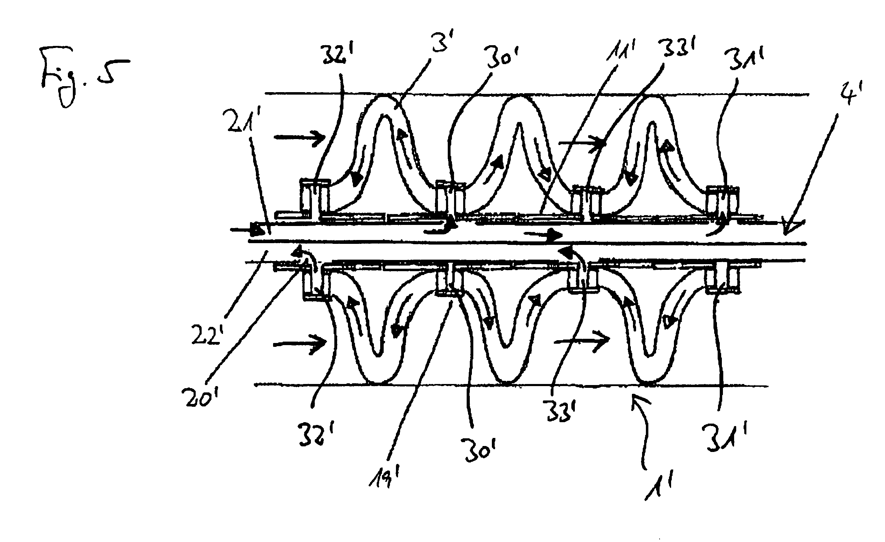

In der alternativen Ausführungsform eines Oxygenators 1' in den Figuren 4 und 5 ist das Faserbündel 3' in mehrere Einheiten (exemplarisch gekennzeichnet mit 3'a und 3'b) getrennt. Zwischen den zwei nachfolgenden Fasereinheiten 3'a, 3'b befinden sich Ringkammern (exemplarisch beziffert mit 19'), an welche die beiden Fasereinheiten 3'a, 3'b angeschlossen sind. Die Entfaltung des Oxygenators 1' erfolgt wie beim Oxygenator 1, denn die mechanische Grundstruktur der beiden Oxygenatoren 1 und 1' ist identisch. Auch die Tordierung des Faserbündels 3'a, 3'b, erfolgt wiederum über eine Verdrehung des Schlauches 9'. Der Katheter 4' ist zweilumig und weist mehrere Öffnungen (exemplarisch beziffert mit 20') an den beiden Lumen 21', 22' auf. Durch Längsverschiebung des Schlauches 9' verschieben sich die Ringkammern 19'. Wenn die Verschiebung abgeschlossen ist, was sich durch Kontakt der Schlitten 11' ergibt, befinden sich die Ringkammern 19' auf der gleichen Höhe wie jeweils zugeordnete Öffnungen 20'. Die Öffnungen 20' treten abwechselnd an den beiden Lumen 21', 22' auf, sodass sich jede zweite Ringkammer 19' mit der Öffnung eines jeweiligen Lumens 21' beziehungsweise 22' deckt. Bei dem gezeigten Ausführungsbeispiel ist der Sauerstoffzufuhrlumen 21' in der Detailansicht der Figur 5 mit zwei Ringkammern 30', 31' verbunden, während der Gasabzuglumen 22' mit zwei Ringkammern 32', 33' durch eine schlitz- oder punktförmige Öfhungsüberdeckung verbunden ist.

Im Betrieb des Oxygenators 1' erfolgt die Sauerstoffversorgung durch den Sauerstoffzufuhrlumen 21' des Katheters 4'. Der Sauerstoff gelangt so in die Ringkammern 30' und 31' und weiter in die Fasern 3', wo der Gasaustausch mit dem Blut stattfindet. Beim Ausströmen aus den Fasern 3' gelangt ein Gasgemisch aus überschüssigem Sauerstoff und dem Blut entnommenen Kohlendioxid in die Ringkammern 32' und 33' und strömt von dort in den Gasabzuglumen 22', durch welchen es aus dem Körper ausströmt.

Die Abdichtung zwischen zwei nebeneinanderliegenden Ringkammern kann auf verschiedene Arten erfolgen, beispielsweise durch Dichtringe. Der Blutdruck selbst kann auch für die Abdichtung genutzt werden, wenn eine elastische Membran den Katheter umgibt und diese Membran durch den höheren Blutdruck gegen den Katheter gedrückt wird und die Gasseite, also den Innenraum, abdichtet.

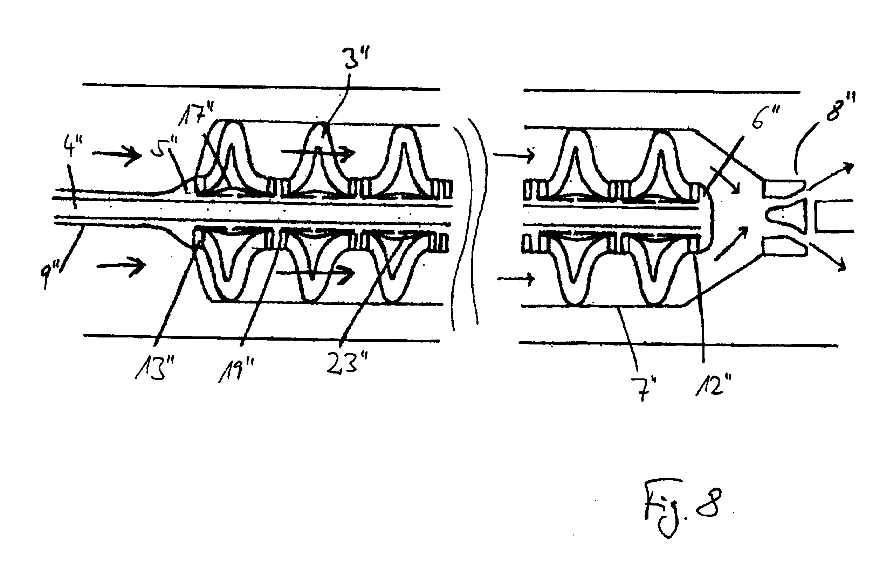

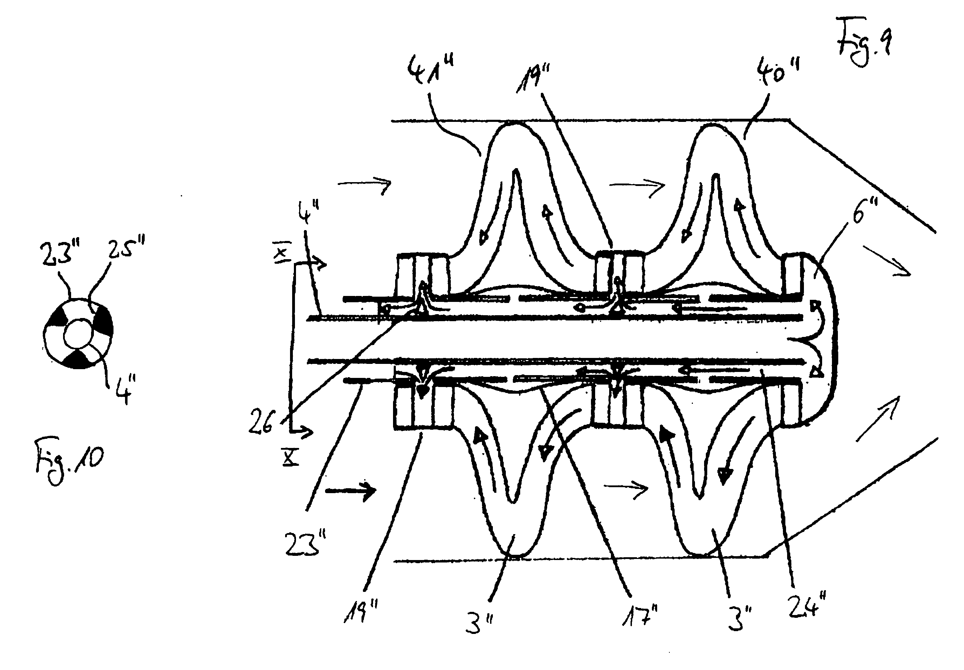

Der weitere Oxygenator 1" in den Figuren 8, 9 und 10 weist ebenfalls mehrere seriell angeordnete Fasereinheiten auf. Der Katheter 4" ist einlumig und ist angeschlossen an die Gaszufuhrkammer 6". Die einzelnen Faserbündel sind mit Schlitten 23" verbunden, welche entlang des Katheters 4" gleiten und rotieren können. Zwischen den Schlitten 23" und dem Katheter befindet sich ein ringförmiger Kanal 24". Die Zentrierung der Schlitten 23" auf dem Katheter 4" erfolgt einfach und sicher durch Vorsprünge 25".

Im Betrieb des Oxygenators 1" strömt Sauerstoff aus der Gaszufuhrkammer 6" teilweise in die Fasern des ersten Faserbündels 3", teilweise aber auch in den ringförmigen Kanal 24" hinein. Benachbarte Schlitten 23" sind durch eine elastisch verformbare Membran 17" verbunden, sodass der Kanal 24" abgedichtet ist. In die Ringkammer 19", welche das erste Faserbündel 40" mit dem zweiten Faserbündel 41" verbindet, gelangt sowohl Sauerstoff aus dem Kanal 24" als auch das Sauerstoff-Kohlendioxid-Gemisch aus dem ersten Faserbündel 40". Die beiden Gasströmungen mischen sich, wobei die Durchmischung durch Strömungsumlenker 26" und die hierdurch entstehenden Turbulenzen und Wirbel forciert wird. Die Strömungsumlenker 26" sind so profiliert, dass das Gas aus dem Kanal 24" die Ringkammer 19" nach Möglichkeit in ihrer ganzen Tiefe erreicht. Durch die Durchmischung sinkt die Kohlendioxidkonzentration des aus den Fasern 40" kommenden Gases entsprechend dem Verhältnis zwischen Gasvolumenstrom im Kanal 24" und in den Fasern 40".

Aus der Kammer 19" strömt das durchmischte Gas wieder teilweise in den Kanal 24" und teilweise in das zweite Faserbündel 41 ". Dieser Prozess wiederholt sich bei jeder Kammer und jedem Faserbündel bis zur abschließenden Gasabzugkammer 5", welche mit dem Schlauch 9" in Verbindung steht. Die Versorgung jeder Kammer 19" mit kohlendioxidärmerem Gasgemisch erhöht die lokalen CO₂-Konzentrationsgradienten zwischen dem Gas in der Faser mit relativ niedrigem Druck und dem Gas im Blut mit relativ hohem Druck, sodass ein deutlich stärkerer Gasaustausch vonstatten geht.

Der Strömungswiderstand für das Gasgemisch im Ringkanal 24" ergibt sich im Wesentlichen durch die Abmessung des Katheters 4" und durch Abmessung und Formgebung der Schlitten 23". Der Gaswiderstand beeinflusst das Verhältnis von im Kanal 24" und im Faserbündel 3" strömendem Gas. Der Druckabfall zwischen den Kammern 5" und 6" wird vom Kanal 24" nicht beeinflusst, weil er im Wesentlichen vom Gaswiderstand in den Fasern 3" abhängt und die Strömung durch die Fasern 3" konstant ist. Der Kanal 24" bewirkt eine Erhöhung des Gesamtvolumenstroms und folglich einen höheren Druckabfall im zentralen Katheter 4". Daher führt der Kanal 24" zu einer besseren Kohlendioxidentsorgung, ohne einen erhöhten Druckabfall in den Fasern 3" zu verursachen. Dies galt bislang als eines der bedeutendsten Probleme bei intravenösen Oxygenatoren. Bei Versuchen haben sich bei den oben beschriebenen Fasern Strömungen von etwa 0,5 l/min durch die Fasern als vorteilhaft herausgestellt, da bei diesen Werten der Druckabfall relativ gering bleibt. Der Druckabfall ist proportional zum Quadrat der Strömungsgeschwindigkeit.

Insbesondere kann das Verhältnis zwischen dem Volumenstrom im freien Kanal und dem Volumenstrom in der Fasern größer als 3, vorzugsweise größer als 4 sein. Besonders gute Werte für den Gasaustausch haben sich bei Verhältniswerten vom etwa 5 ergeben.

Es sei betont, dass ein Kanalsystem, welches mittels eines freien Kanals über Mischkammern die Faserbündel mit kohlendioxidärmerem Gas insbesondere mit den angegebenen Volumenstromverhältnissen versorgt, auch für sich genommen und unabhängig von sämtlichen anderen vorgeschlagenen Merkmalen vorteilhaft ist.

Selbstverständlich ist es auch möglich, Merkmale der gezeigten Ausführungsbeispiele miteinander zu kombinieren. Beispielsweise kann in einem weiteren Oxygenator eine gemeinsame Gasversorgung für über die Länge des Oxygenators durchlaufende und im Verlauf unterteilte Fasern vorgesehen sein. Auch können beispielsweise die Längen der einzelnen Abschnitte unterschiedlich sein.

Insbesondere kann die Pumpe an der zuerst angeströmten Seite des Oxygenators liegen. Hierfür müsste die undurchlässige Hülle dort dicht an die Pumpe angeschlossen sein, um eine Strömung vom Rotoreinlauf zum Rotorauslauf nur über den Rotor zuzulassen. Vorteilhaft kann ein dergestalt konstruierter Oxygenator nach dem Einsatz besser wieder aus der Hohlvene herausgezogen werden, weil sich die Hülle beim Entnehmen einfach flach an den Oxygenator anlegt.

Außerdem ist bei einem Oxygenator, welcher die Pumpe im angeströmten Ende einer undurchlässigen Hülle trägt, der Blutdruck innerhalb der Hülle größer als im physiologischen Kreislauf. Hierdurch entsteht eine resultierende Kraft, welche auf die Hülle radial nach außen wirkt. Diese Kraft kann dazu genutzt werden, die Hülle auf ihren im Einsatz benötigten Durchmesser aufzuweiten.

Wenn die Pumpeneinheit am angeströmten Ende des Oxygenators liegt, stellt sich die Aufgabe, möglichst raumsparend die Pumpe, die Gaszuführung und die Gasrückführung anzuordnen. Um den Oxygenator in seiner Längserstreckung möglichst kurz zu halten, empfiehlt es sich, die hierfür nötigen Bauteil in einem Querschnitt anzuordnen.

Hierzu wird eine gemeinsame Patrone vorgeschlagen, in welcher ein Katheter und vorzugsweise auch die Pumpe ausmittig angeordnet sind. Die Pumpe benötigt im Regelfall einen größeren Querschnitt als ein Gaskatheter. Wenn ein Katheter mittig im Querschnitt des Oxygenators angeordnet ist und eine Pumpe neben dem Katheter liegt, dann wird an dieser Stelle bereits ein Radius des Oxygenators in Höhe des halben Katheterdurchmessers plus des gesamten Pumpendurchmessers benötigt. Wenn die Pumpe mittig liegt und ein Katheter seitlich der Pumpe geführt ist, so errechnet sich der benötigte Oxygenatorradius zum halben Pumpendurchmesser plus des Katheterdurchmessers. Durch die ausmittige Anordnung des Katheters ergibt sich somit ein Querschnittsvorteil, sobald die Pumpe einen größeren Durchmesser hat als der Katheter.

Die Pumpeneinheit des Oxygenators kann besonders platzsparend ausgeführt werden, indem der Katheter und die Pumpe beide ausmittig so angeordnet sind, dass die Längsachse des Oxygenators auf einer Verbindungslinie zwischen der Längsachse der Pumpe und der Längsachse des Katheters liegen. Die Pumpe kann dabei insbesondere mit ihrem Umfang am Umfang des Oxygenators an der Pumpeneinheit, beispielsweise also an der Wandung einer gemeinsamen Patrone für Pumpe und Katheter, anliegen.

An eine Patrone kann die undurchlässige Hülle des Oxygenators unmittelbar angeschlossen werden. Zur Befestigung eignet sich besonders eine zylindrische Patrone.

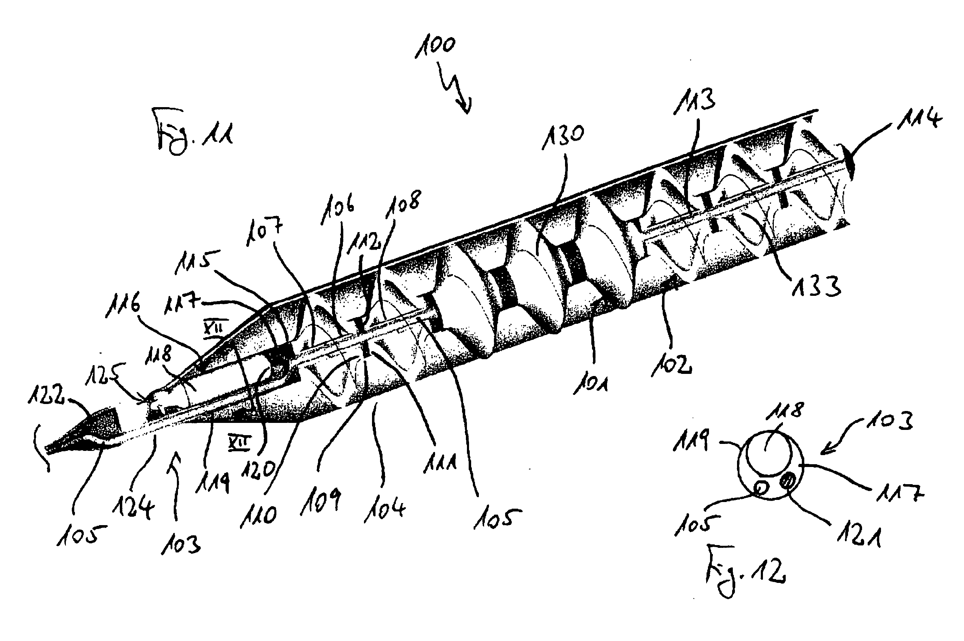

Eine solche Ausgestaltung liegt im Ausführungsbeispiel der Figuren 11 und 12 vor. Der Oxygenator 100 besteht im Wesentlichen aus acht seriell verbundenen Faserbündeln (exemplarisch beziffert mit 101, 102), welche gemeinsam mit der Pumpeneinheit 103 in einer undurchlässigen Hülle 104 angeordnet sind.

Die Faserbündel werden entlang eines Sauerstoffkatheters 105 auf Faserhaltern (exemplarisch beziffert mit 106, 107, 108) gehalten und mit diesen geführt. Zwei benachbarte Faserhalter 106, 108 unterschiedlicher Faserbündel sind gegeneinander unverdrehbar und nicht längs verlagerbar verbunden durch eine zylindrische Hülse (exemplarisch beziffert mit 109), welche mit vergossenen Anschlüssen (exemplarisch beziffert mit 110, 111) beispielsweise klemmend oder verklebt verbunden ist. Zwischen den vergossenen Anschlüssen 110, 111 und der Hülse 109 ergibt sich eine Mischkammer 112.

Die Mischkammer 112 steht an ihrer Innenseite gleichzeitig mit einem kreisringförmigen Mischkanal 113 in Verbindung. Der Mischkanal 113 verläuft von einer Sauerstoffspeisekammer 114 zu einem Abzuganschluss 115 einer Zylinderpatrone 116 zwischen dem Sauerstoffkatheter 105 und den Faserhaltern 106, 107, 108 ohne Unterbrechung. Am Abzuganschluss 115 geht der Mischkanal 113 in eine Hohlkammer 117 über. In der Hohlkammer 117 sind der Sauerstoffkatheter 105 und eine Pumpe 118 ausmittig angeordnet, wobei die Pumpe 118 an einer Patronenwand 119 unmittelbar anliegt und dort von einer Trennkammer (nicht beziffert) fixiert ist. Die Trennkammer ist gegenüber der Hohlkammer 117 abgedichtet. Eine Ausnahme liegt lediglich an einer Kabeldurchführung 120 vor. Durch die Kabeldurchführung 120 verläuft ein Stromkabel 121 von der Pumpe 118 in die Hohlkammer 117 und von dort mit dem Sauerstoffkatheter 105 durch einen Schlauch 122. Die Hohlkammer 117 ist mit dem Schlauch 122 verbunden. Die Pumpe 118 ist jedoch an ihrer der Kabeldurchführung 120 zugewandeten Seite im Wesentlichen kegelförmig ausgebildet und dichtet die Durchführung 120 selbst ab. Die Patrone 116 liegt koaxial mit der Längsachse des Oxygenators 100 und ist - bis auf eine Verbindung 124 der Hohlkammer 117 zum Rückführen von kohlendioxidangereichertem Gas - zweigeteilt, sodass ein Pumpeneinlauf 125 frei liegt zum Anströmen mit Blut.

Benachbarte Faserhalter 106, 107 desselben Faserbündels sind gegeneinander um 240 ° verdreht. Die Faserbündel aus 35 mm langen Fasern sind entsprechend tordiert (eine Faser pro Bündel ist gekennzeichnet, exemplarisch beziffert mit 130). Die Faserhalter liegen formschlüssig aneinander und weisen an der Stoßringfläche eine Nut 131 beziehungsweise eine Nase 132 auf, sodass sie sich nicht entdrehen können, solange sie nicht so weit auseinander bewegt werden, dass die Nut die Nase freigibt. Im Inneren der Faserbündel sind die Faserhalter 106, 107 zudem mit einer elastischen Membran (exemplarisch beziffert mit 133) verbunden. Die Membran 133 dichtet den Mischkanal 113 gegen den für Blut vorgesehenen Strömungsraum zwischen den Faserhaltern 106, 107, 108 und der Hülle des Oxygenators 100 ab. Am angeströmten Ende des Oxygenators 100 ist die Hülle 104 dicht mit der Patrone 116 verbunden, sodass an dieser Seite ein Einströmen von Blut nur durch den Pumpeneinlauf 125 erfolgen kann.

Im Betrieb des Oxygenators 100 wird durch die Pumpe 118 ein Überdruck innerhalb der Hülle 104 erzeugt. Der Gasdruck muss immer kleiner sein als der Blutdruck, daher kann bei erhöhtem Blutdruck auch der Gasdruck entsprechend höher ausgelegt werden. Bei Versuchen hat sich allein hierdurch ein um etwa ein Zehntel gesteigerter Gasaustausch ergeben.

Durch die Anordnung der Pumpeneinheit 103 am angeströmten Ende ist es zudem möglich, das stromabwärts liegende Ende der Hülle 104 ohne Befestigung auszuführen. Durch den Blutdruck wird die Hülle 104 von selbst radial ausgedehnt und durch die Blutströmrichtung längs gestreckt. Infolge der einfachen Hüllenstruktur kann der Oxygenator nach dem Einsatz der Hohlvene leichter wieder entnommen werden.

## Patentansprüche

1. Intravenöser Oxygenator (1) zum Einführen in eine Vene, mit einem Faserbündel (3) sauerstoff- und kohlendioxiddurchströmbarer Fasern, wobei die Fasern jeweils mit einem ersten Anschluss (12) an eine Gaszufuhr angeschlossen sind und mit einem zweiten Anschluss (13) an einen Gasabzug angeschlossen sind, sodass Sauerstoff und Kohlendioxid von den ersten Anschlüssen durch die Fasern zu den zweiten Anschlüssen strömen kann, wobei die Fasern mit einem ersten und einem zweiten Faserhalter mit verbunden und die Anschlüsse entlang einer Längsachse des Oxygenators verlagerbar sind, ***dadurch gekennzeichnet, dass*** die Faserhalter (11) gegeneinander verdrehbar um die Längsachse des Oxygenators und längs dieser Achse verlagerbar gelagert sind, und weiterhin **kennzeichnet durch** ein radial verformbares Gehäuse (7) mit einer undurchlässigen Hülle.

2. Intravenöser Oxygenator nach Anspruch 1, ***gekennzeichnet durch*** einen ersten Mitnehmer am ersten Faserhalter und einen zweiten Mitnehmer am zweiten Faserhalter, wobei die Mitnehmer zueinander gerichtet sind und bei Presskontakt der beiden Faserhalter (11) gegeneinander eine relative Verdrehung des ersten Faserhalters gegen den zweiten Faserhalter zumindest in eine Drehrichtung nur bis zu einer Drehgrenze ohne Mitnahme des zweiten Faserhalters zulassen.

3. Intravenöser Oxygenator nach Anspruch 2, ***dadurch gekennzeichnet, dass*** die Drehgrenze bei einer relativen Verdrehung von 90° bis 300°, vorzugsweise von 150° bis 270°, besonders bevorzugt von etwa 240°, pro 30 mm Faserlänge zwischen den beiden Faserhaltern liegt.

4. Intravenöser Oxygenator nach einem der vorhergehenden Ansprüche, ***gekennzeichnet* durch** eine Anschlageinrichtung an den Faserhaltern (11) zur Begrenzung einer Verlagerung der Anschlüsse zueinander.

5. Intravenöser Oxygenator nach einem der vorhergehenden Ansprüche, ***dadurch gekennzeichnet, dass*** die Faserhalter (11) im Innenraum des Faserbündels angeordnet sind.

6. Intravenöser Oxygenator nach einem der vorhergehenden Ansprüche, ***gekennzeichnet durch*** eine im Wesentlichen elastische Verbindung zwischen den zwei Faserhaltern, insbesondere eine Membran (17) und/oder eine Linearfeder.

7. Intravenöser Oxygenator nach einem der vorhergehenden Ansprüche, ***gekennzeichnet durch*** eine Spiralführung der Faserhalter (11) entlang der Längsachse des Oxygenators.

8. Intravenöser Oxygenator nach einem der vorhergehenden Ansprüche, ***gekennzeichnet durch*** eine Blutpumpe (8) zum Fördern von Blut **durch** das Faserbündel.

9. Intravenöser Oxygenator nach einem der vorhergehenden Ansprüche, ***dadurch gekennzeichnet, dass*** das Gehäuse (7) einen Durchmesser von höchstens 30 mm, insbesondere von höchstens 25 mm, annehmen kann.

10. Intravenöser Oxygenator nach Anspruch 9, ***gekennzeichnet durch*** ein Drahtgitter als tragende Struktur des Gehäuses (7).

## Claims

1. An intravenous oxygenator (1) for insertion into a vein, comprising a fibre bunch (3) with fibres through which oxygen and carbon dioxide can flow, wherein in each case the fibres by way of a first connection (12) are connected to a gas supply, and by way of a second connection (13) are connected to a gas vent so that oxygen and carbon dioxide can flow from the first connections through the fibres to the second connections, wherein the fibres are connected to a first and a second fibre holder and the connections are displaceable along a longitudinal axis of the oxygenator, **characterised in that** the fibre holders (11) are held so as to be rotatable in relation to each other on the longitudinal axis of the oxygenator and so as to be displaceable along this axis, and further **characterised by** a radially deformable housing (7) comprising an impermeable envelope.

2. The intravenous oxygenator according to claim 1, **characterised by** a first catch on the first fibre holder and a second catch on the second fibre holder, wherein the catches are facing each other and in the case of press contact of the two fibre holders (11) against each other permit relative rotation of the first fibre holder in relation to the second fibre holder at least in one direction of rotation only to a limit of rotation without catching the second fibre holder.

3. The intravenous oxygenator according to claim 2, **characterised in that** the limit of rotation at a relative rotation of 90° to 300°, preferably of 150° to 270°, particularly preferably of approximately 240°, for each 30 mm of fibre length is between the two fibre holders.

4. The intravenous oxygenator according to any one of the preceding claims, **characterised by** an end stop device on the fibre holders (11) to limit displacement of the connections relative to each other.

5. The intravenous oxygenator according to any one of the preceding claims, **characterised in that** the fibre holders (11) are arranged in the interior of the fibre bunch.

6. The intravenous oxygenator according to any one of the preceding claims, **characterised by** an essentially elastic connection between the two fibre holders, in particular a membrane (17) and/or a linear spring.

7. The intravenous oxygenator according to any one of the preceding claims, **characterised by** a helical arrangement of the fibre holders (11) along the longitudinal axis of the oxygenator.

8. The intravenous oxygenator according to any one of the preceding claims, **characterised by** a blood pump (8) for conveying blood through the fibre bunch.

9. The intravenous oxygenator according to any one of the preceding claims, **characterised in that** the housing (7) can assume a diameter of a maximum of 30 mm, in particular of a maximum of 25 mm.

10. The intravenous oxygenator according to claim 9, **characterised by** a wire grating as a supporting structure of the housing (7).

## Revendications

1. Oxygénateur intraveineux (1) à introduire dans une veine, comportant un faisceau de fibres (3) composé de fibres où peuvent passer de l'oxygène et du dioxyde de carbone, les fibres étant raccordées respectivement par un premier raccordement (12) à un système d'alimentation en gaz et par un deuxième raccordement (13) à un système d'extraction de gaz, de sorte que l'oxygène et le dioxyde de carbone peuvent s'écouler à travers les fibres depuis les premiers raccordements vers les deuxièmes raccordements, les fibres étant raccordées respectivement par un premier et un deuxième support de fibres et les raccordements pouvant être déplacés le long d'un axe longitudinal de l'oxygénateur, **caractérisé en ce que** les supports de fibres (11) s'appuient de manière à pouvoir tourner l'un par rapport à l'autre autour de l'axe longitudinal de l'oxygénateur et le long de cet axe, et **caractérisé en outre par** un boîtier déformable radialement (7) à enveloppe imperméable.

2. Oxygénateur intraveineux selon la revendication 1, **caractérisé par** un premier entraîneur au niveau du premier support de fibres et un deuxième entraîneur au niveau du deuxième support de fibres, les entraîneurs étant dirigés l'un vers l'autre et permettant, en cas de contact de compression des deux supports de fibres (11) l'un contre l'autre, une rotation relative du premier support de fibres par rapport au deuxième support de fibres au moins dans un sens de rotation seulement jusqu'à une limite de rotation ou jusqu'à un entraînement du deuxième support de fibres.

3. Oxygénateur intraveineux selon la revendication 2, **caractérisé en ce que** la limite de rotation, dans le cas d'un mouvement relatif de 90° à 300°, de préférence 150° à 270°, de manière particulièrement préférentielle d'environ 240°, par longueur de fibre de 30 mm, se situe entre les deux supports de fibres.

4. Oxygénateur intraveineux selon une des revendications précédentes, **caractérisé par** un système de butée placé sur les supports de fibres (11) afin de limiter un décalage des raccordements l'un par rapport à l'autre.

5. Oxygénateur intraveineux selon une des revendications précédentes, **caractérisé en ce que** les supports de fibres (11) sont disposés à l'intérieur du faisceau de fibres.

6. Oxygénateur intraveineux selon une des revendications précédentes, **caractérisé par** un raccordement sensiblement élastique entre deux supports de fibres, notamment une membrane (17) et/ou un ressort linéaire.

7. Oxygénateur intraveineux selon une des revendications précédentes, **caractérisé par** un guidage en spirale des supports de fibres (11) le long de l'axe longitudinal de l'oxygénateur.

8. Oxygénateur intraveineux selon une des revendications précédentes, **caractérisé par** une pompe à sang (8) permettant le transport du sang à travers le faisceau de fibres.

9. Oxygénateur intraveineux selon une des revendications précédentes, **caractérisé en ce que** le boîtier (7) peut prendre un diamètre de 30 mm au maximum, en particulier de 25 mm au maximum.

10. Oxygénateur intraveineux selon la revendication 9, **caractérisé par** une grille en fil métallique servant de structure porteuse du boîtier (7).
